(19) Europäisches Patentamt
European Patent Office
Office européen des brevets

(11) Veröffentlichungsnummer: **0 108 037**
**B1**

(12) # EUROPÄISCHE PATENTSCHRIFT

(45) Veröffentlichungstag der Patentschrift: **07.06.89**

(21) Anmeldenummer: **83810430.5**

(22) Anmeldetag: **26.09.83**

(51) Int. Cl.⁴: **C 08 F 2/50**, G 03 C 1/68, C 09 D 3/74, C 09 D 11/10, C 07 C 49/84, C 07 C 49/83, C 07 D317/26

(54) **Propiophenonderivate als Photoinitiatoren für die Photopolymerisation.**

(30) Priorität: **01.10.82 CH 5807/82**

(43) Veröffentlichungstag der Anmeldung: **09.05.84 Patentblatt 84/19**

(45) Bekanntmachung des Hinweises auf die Patenterteilung: **07.06.89 Patentblatt 89/23**

(84) Benannte Vertragsstaaten: **BE DE FR GB IT NL**

(56) Entgegenhaltungen:
**FR-A- 2 209 789**
**FR-A- 2 348 228**
**US-A- 3 801 329**
**US-A- 4 318 791**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Rutsch, Werner, Dr.**
**Avenue Weck-Reynold 1**
**CH-1700 Fribourg (CH)**
Erfinder: **Kirchmayr, Rudolf, Dr.**
**Ettingerstrasse 9**
**CH-4147 Aesch (CH)**
Erfinder: **Hüsler, Rinaldo, Dr.**
**Belchenstrasse 12**
**CH-4054 Basel (CH)**
Erfinder: **Dietliker, Kurt, Dr.**
**Ave Jean-Marie Musy 6**
**CH-1700 Fribourg (CH)**

**EP 0 108 037 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Verwendung ausgewählter aromatischaliphatischer Ketone als Initiatoren für die Photopolymerisation äthylenisch ungesättigter Verbindungen oder die photochemische Vernetzung von Polyolefinen sowie die solche Initiatoren enthaltenden photopolymerisierbaren bzw. vernetzbaren Systeme.

Photochemische Polymerisationsprozesse haben in der Technik erhebliche Bedeutung erlangt, vor allem in solchen Fällen, wo dünne Schichten in kurzer Zeit gehärtet werden müssen, wie z. B. bei der Härtung von Lacküberzügen oder bei Trocknung von Druckfarben. Die UV-Bestrahlung in Gegenwart von Photoinitiatoren zeigt gegenüber herkömmlichen Härtungsverfahren eine Reihe von Vorteilen, deren wichtigster wohl die hohe Geschwindigkeit der Photohärtung ist. Die Geschwindigkeit ist stark vom verwendeten Photoinitiator abhängig, und es hat nicht an Versuchen gefehlt, die herkömmlichen Initiatoren durch immer bessere und wirksamere Verbindungen zu ersetzen.

Zu den wichtigsten Photoinitiatoren gehören Derivate aromatisch-aliphatischer Ketone, vor allem Propiophenonderivate, wie sie beispielsweise in der US-PS 4 318 791, in der US-PS 4 072 694 und in der DE-OS 2 357 866 beschrieben werden.

Derartige Verbindungen sind jedoch insbesondere in bezug auf ihre Lagerbeständigkeit, ihre Reaktivität und ihre Neigung zur Vergilbung noch nicht optimal.

In der Technik besteht daher ein Bedarf an Photoinitiatoren, die im Substrat gut löslich sind und bei guter Dunkellagerstabilität die Photopolymerisation rascher auslösen und eine noch höhere Polymerausbeute pro Zeiteinheit ergeben als die bekannten Photoinitiatoren. Durch die Verwendung solcher verbesserter Photoinitiatoren würden sich die kostspieligen industriellen UV-Bestrahlungsanlagen besser ausnützen lassen.

Beispielsweise trotz der Lehre der US-PS 4 318 791, die eine Vielzahl von Propiophenonderivaten generisch umfasst, konnten die festgestellten Mängel damit nicht hinreichend behoben werden.

Es wurde gefunden, dass bestimmte aromatisch-aliphatische Propiophenone ausgezeichnete Eigenschaften als Photoinitiatoren besitzen und dadurch die Photopolymerisation äthylenisch ungesättigter Verbindungen verbessern.

Die Erfindung betrifft somit die Verwendung ausgewählter Propiophenone der Formel I

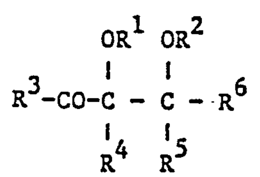

(I)

worin

$R^1$ und $R^2$ zusammen ein $C_3$-$C_6$ Alkyliden- oder ein mit Hydroxy, $C_1$-$C_4$-Alkoxy oder Phenyl substituiertes $C_2$-$C_6$-Alkyliden-, ein geradkettiges oder verzweigtes $C_2$-$C_6$ Alkadiyl-, Cyclopentyliden-, Cyclohexyliden-, ein 2,2,2-Trichloräthyliden-, 2-Furylmethyliden- oder Dimethylsilyliden-Radikal,

$R^3$ unsubstituiertes oder beispielsweise durch einen oder mehrere Reste —Cl, $C_1$-$C_4$ Alkyl, $C_1$-$C_4$ Alkoxy oder durch $C_1$-$C_4$ Alkylthio substituiertes Phenyl, ferner Benzoylphenyl, Phenoxyphenyl oder Phenylthiophenyl,

$R^4$ $C_1$-$C_4$ Alkyl, und

$R^5$ Wasserstoff oder $C_1$-$C_4$ Alkyl bedeuten, oder ferner

$R^4$ und $R^5$ zusammen ein Tri- oder Tetramethylen-Radikal darstellen,

und

$R^6$ Wasserstoff, $C_1$-$C_4$ Alkyl, —$CCl_3$ oder Phenyl bedeutet.

Als Initiatoren für die Photopolymerisation äthylenisch ungesättigter Verbindungen sowie für die photochemische Vernetzung von Polyolefinen.

Stellen $R^4$, $R^5$ und $R^6$ $C_1$-$C_4$ Alkyl dar, so handelt es sich um geradkettige oder verzweigte Alkylgruppen wie Methyl, Aethyl, n-Propyl, Isopropyl, n-Butyl, sec.-Butyl und tert.-Butyl, insbesondere aber um Methyl.

Ist Phenyl als $R^3$ durch $C_1$-$C_4$ Alkyl substituiert, so handelt es sich beispielsweise um Methyl-, Aethyl-, n-Propyl-, Isopropyl-, n-Butyl- oder tert.-Butyl-Substituenten.

Ist Phenyl als $R^3$ durch $C_1$-$C_4$ Alkoxy oder durch $C_1$-$C_4$ Alkylthio substituiert, so handelt es sich beispielsweise um Methoxy-, Aethoxy-, Propoxy- oder tert.-Butoxygruppen resp. um Methyl-, Aethyl-, Propyl- oder tert. Butyl-thio-Substituenten.

Stellen $R^1$ und $R^2$ zusammen ein $C_3$-$C_6$ Alkylidenradikal dar, so handelt es sich um Propyliden oder insbesondere um Isopropyliden, stellen sie zusammen ein geradkettiges oder verzweigtes $C_2$-$C_6$ Alkadiyl dar, so handelt es sich beispielsweise um Aethandiyl oder Methyläthandiyl.

Stellen $R^1$ und $R^2$ zusammen ein mit Hydroxy substituiertes $C_2$-$C_6$-Alkylidenradikal dar, so handelt es sich bevorzugt um 1-Hydroxy-2-propyliden, stellen sie zusammen ein mit $C_1$-$C_4$ Alkoxy substituiertes $C_2$-

$C_6$-Alkylidenradikal dar, so ist dieses vorzugsweise 1-Methoxy-2-propyliden, stellen sie zusammen ein mit Phenyl substituiertes $C_2$-$C_6$-Alkylidenradikal dar, so handelt es sich insbesondere um 1-Phenyl-1-äthyliden.

Bevorzugt sind Verbindungen der Formel I, worin $R^3$ Phenyl, $R^4$ $C_1$-$C_4$ Alkyl und $R^5$ Wasserstoff bedeuten, oder worin $R^1$ und $R^2$ zusammen Isopropyliden bedeuten, oder $R^4$ und $R^5$ zusammen ein Tri- oder Tetramethylen-Radikal darstellen, und $R^6$ Wasserstoff ist.

Bevorzugt sind ebenso Verbindungen der Formel I, worin $R^1$ und $R^2$ zusammen ein Dimethylsilyliden-Radikal bedeuten.

Besonders bevorzugt sind Verbindungen der Formel I, worin $R^5$ und $R^6$ Wasserstoff, $R^4$ Methyl und $R^3$ Phenyl bedeuten, oder worin $R^1$ und $R^2$ zusammen Isopropyliden bedeuten.

Insbesondere bevorzugt sind Verbindungen der Formel I, worin $R^1$ und $R^2$ zusammen Isopropyliden oder Dimethylsilyliden, $R^3$ Phenyl, $R^4$ Methyl, $R^5$ Wasserstoff oder $R^4$ und $R^5$ zusammen ein Tetramethylen-Radikal und $R^6$ Wasserstoff oder Phenyl bedeuten.

Beispiele für einzelne Verbindungen der Formel I sind :

4-Benzoyl-5-trichlormethyl-2,2,4-trimethyl-1,3-dioxolan
4-Benzoyl-2,2,4-trimethyl-1,3-dioxolan
4-Benzoyl-4-methyl-2,2-pentamethylen-1,3-dioxolan
4-Aethyl-4-benzoyl-2,2-tetramethylen-1,3-dioxolan
4-Aethyl-4-(4-methoxybenzoyl)-2-propyl-1,3-dioxolan
4-Benzoyl-2,4-dimethyl-2-äthyl-5-phenyl-1,3-dioxolan
4-Aethyl-4-benzoyl-2-isopropyl-5-phenyl-1,3-dioxolan
2-Benzoyl-2-methyl-3-phenyl-1,4-dioxolan
1-Benzoyl-8,8-dimethyl-7,9-dioxabicyclo[4.3.0]nonan
1-Benzoyl-8,8-pentamethylen-7,9-dioxabicyclo[4.3.0]nonan

Die Verbindungen der Formel I sind neu und stellen daher auch einen Gegenstand der vorliegenden Erfindung dar. Die Herstellung erfolgt in Analogie zu den bekannten Verbindungen, so beispielsweise gemäss den in US-PS 4 318 791 und US-PS 4 072 694 angegebenen relevanten Verfahren.

Als besonders vorteilhaft hat sich der folgende Syntheseweg erwiesen :

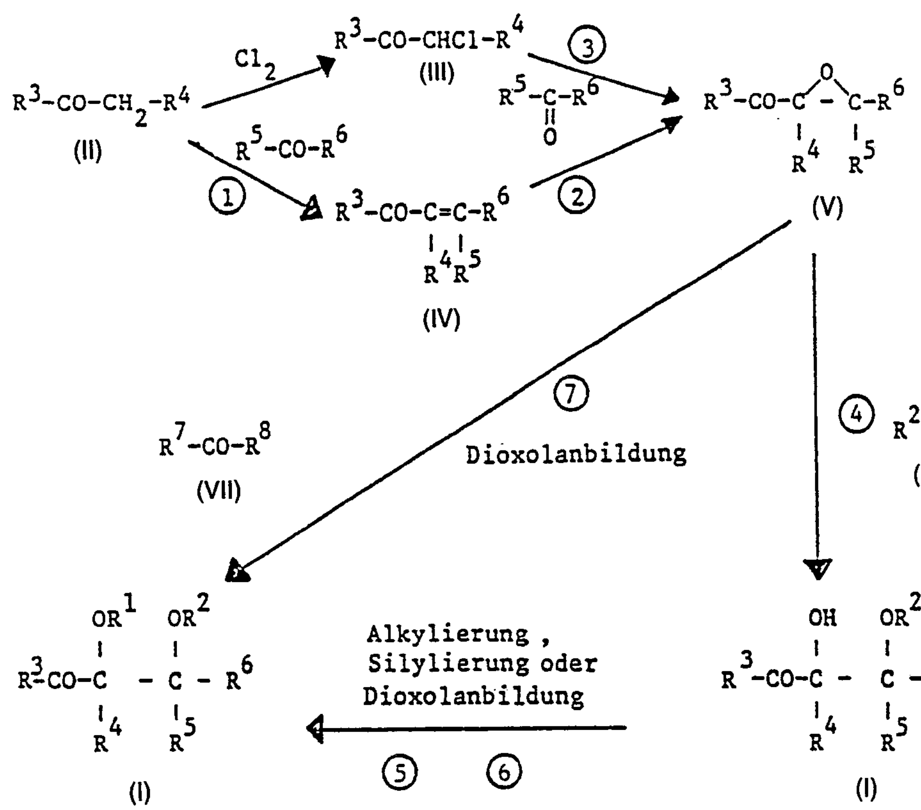


Ausgehend von einem Propiophenonderivat der Formel II wird die Verbindung der Formel V entweder mittels $Cl_2$ über die chlorierte Verbindung der Formel III oder über die Verbindung der Formel IV hergestellt, welche nach Reaktion mit einem entsprechenden Alkohol der Formel VI bzw. mit $H_2O$ zur erfindungsgemässen Verbindung der Formel I und nach weiterer Alkylierung, Silylierung oder Dioxolanbildung zur erfindungsgemässen Verbindung der Formel I umgesetzt wird. Verbindungen der Formel I können auch direkt aus Verbindungen der Formel V durch Dioxolanbildung mit einem Keton oder Aldehyd der Formel VII gebildet werden. Dabei stellen $R^7$ Wasserstoff, $C_1$-$C_5$ Alkyl oder Phenyl, $R^8$

Wasserstoff oder $C_1$-$C_2$ Alkyl und $R^7$ und $R^8$ zusammen ein Tetramethylen- oder Pentamethylen-Radikal dar. In den Formeln I, II, III, IV, V und VI haben die Reste $R^1$ bis $R^6$ die oben angegebene Bedeutung.

Die eingekreisten Nummern geben die Literaturstellen an, in welchen derartige analoge Verfahren beschrieben sind :

① J. Chem. Soc., 79, 928 (1901), ② J. Org. Chem., 28, 250 (1963) oder Org. Syntheses, 55, 52 (1976), ③ J. Am. Chem. Soc., 75, 2042 (1953), ④ J. Chem. Soc., 73, 3293 (1958) oder Houben-Weyl, Methoden der organischen Chemie, Bd. VI/3, 40-44, 456-457 (1965), ⑤ T.W. Greene, Protective Groups in Organic Synthesis, S. 40-42 (1981) oder Houben-Weyl, Methoden der organischen Chemie, Bd. VI/3, 229-232 (1963), ⑥ J. Am. Chem. Soc. 92, 5394 (1970), ⑦ Khim. Geterotsikl. Soedin 1975, 907.

Ein weiterer Gegenstand der Erfindung ist eine photopolymerisierbare Zusammensetzung enthaltend mindestens eine äthylenisch ungesättigte photopolymerisierbare Verbindung und eine wirksame Menge eines erfindungsgemässen Propiophenonderivates der Formel I.

Solche Verbindungen sind beispielsweise ungesättigte Monomere wie Ester von Acryl- oder Methacrylsäure, z. B. Methyl-, Aethyl-, n- oder tert.-Butyl-, Isooctyl- oder Hydroxyäthylacrylat, Methyl- oder Aethyl-methacrylat, Aethylendiacrylat, Neopentyl-diacrylat, Trimethylolpropantrisacrylat, Pentaerythrittetraacrylat oder Pentaerythrittrisacrylat ; Acrylnitril, Methacrylnitril, Acrylamid, Methacrylamid, N-substituierte (Meth)acrylamide ; Vinylester wie z. B. Vinylacetat, -propionat, -acrylat oder -succinat ; sonstige Vinylverbindungen wie Vinyläther, Styrol, Alkylstyrole, Halogenstyrole, Divinylbenzol, Vinylnaphthalin, N-Vinylpyrrolidon, Vinylchlorid oder Vinylidenchlorid ; Allylverbindungen wie Diallylphthalat, Diallylmaleat, Triallylisocyanurat, Triallylphosphat oder Aethylenglykol-diallyläther und die Mischungen von solchen ungesättigten Monomeren.

Photopolymerisierbare Verbindungen sind weiterhin ungesättigte Oligomere oder Polymere und deren Mischungen mit ungesättigten Monomeren. Hierzu zählen thermoplastische Harze, die ungesättigte Gruppen wie Fumarsäureester, Allylgruppen oder Acrylat- oder Methacrylatgruppen enthalten. Meist sind diese ungesättigten Gruppen über funktionelle Gruppen an die Hauptkette dieser linearen Polymeren gebunden. Grosse Bedeutung haben Gemische von Oligomeren mit einfach und mehrfach ungesättigten Monomeren. Beispiele für solche Oligomere sind ungesättigte Polyester, ungesättigte Acrylharze und Isocyanat- oder Epoxid- modifizierte Acrylatoligomere sowie Polyätheracrylatoligomere. Beispiele für mehrfach ungesättigte Verbindungen sind vor allem die Acrylate von Diolen und Polyolen, z. B. Hexamethylen-diacrylat oder Pentaerythrit-tetraacrylat. Auch als einfach ungesättigte Monomere werden Acrylate bevorzugt wie z. B. Butylacrylat, Phenylacrylat, Benzylacrylat, 2-Aethyl-hexylacrylat oder 2-Hydroxypropylacrylat. Man hat es durch Auswahl aus den verschiedenen Vertretern der drei Komponenten in der Hand, die Konsistenz des unpolymerisierten Gemisches sowie die Plastizität des polymerisierten Harzes zu variieren.

Neben diesen Dreikomponenten-Gemischen spielen bei den Polyesterharzen vor allem Zweikomponenten-Gemische eine grosse Rolle. Diese bestehen meist aus einem ungesättigten Polyester und einer Vinylverbindung. Die ungesättigten Polyester sind oligomere Veresterungsprodukte mindestens einer ungesättigten Dicarbonsäure wie z. B. Malein-, Fumar- oder Citraconsäure, und meist mindestens einer gesättigten Dicarbonsäure, wie z. B. Phthalsäure, Bernsteinsäure, Sebazinsäure oder Isophthalsäure, mit Glykolen wie z. B. Aethylenglykol, Propandiol-1,2, Di- oder Triäthylenglykol oder Tetramethylenglykol, wobei zur Modifizierung meist auch Monocarbonsäuren und Monoalkohole mitverwendet werden. Diese ungesättigten Polyester werden üblicherweise in einer Vinyl- oder Allylverbindung gelöst, bevorzugt wird hierfür Styrol verwendet.

Bevorzugt als äthylenisch ungesättigte photopolymerisierbare Verbindungen sind Mono-, Di- oder polyfunktionelle Acrylatester und/oder Methacrylatester sowie Mischungen davon.

Zahlreiche der erfindungsgemäss verwendbaren Verbindungen sind auch als Initiatoren in wässrigen photopolymerisierbaren bzw. vernetzbaren Systemen geeignet. Beispiele für wässrige Dispersionen sind beschrieben in EP 12339, EP 21078, EP 41125, DE-OS 2 936 039, DE-OS 3 005 036.

Photopolymerisierbare Systeme, wie sie für die verschiedenen Zwecke verwendet werden, enthalten meist ausser den photopolymerisierbaren Verbindungen und dem Photoinitiator eine Reihe sonstiger Zusätze. So ist es vielfach üblich, thermische Inhibitoren zuzusetzen, die vor allem während der Herstellung der Systeme durch Mischen der Komponenten vor einer vorzeitigen Polymerisation schützen sollen. Hierzu werden beispielsweise Hydrochinon, Hydrochinonderivate, p-Methoxyphenol, β-Naphthylamin oder β-Naphthole verwendet. Weiter können geringe Mengen von UV-Absorbern zugesetzt werden wie z. B. solche vom Benztriazol-, Benzophenon- oder Oxalanilid-Typ. Ebenso lassen sich Lichtschutzmittel vom Typus sterischgehinderter Amine (HALS) zusetzen.

Zur Erhöhung der Dunkellagerstabilität können Kupferverbindungen wie Kupfernaphthenat, -stearat, oder -octoat, Phosphorverbindungen wie Triphenylphosphin, Tributylphosphin, Triäthylphosphit, Triphenylphosphit oder Tribenzylphosphit, quaternäre Ammoniumverbindungen wie Tetramethylammoniumchlorid oder Trimethyl-benzylammoniumchlorid oder Hydroxylaminderivate, wie z. B. N-Diäthylhydroxylamin, zugesetzt werden. Weiterhin können die photopolymerisierbaren Systeme Kettenübertragungsmittel wie z. B. N-Methyldiäthanolamin, Triäthanolamin oder Cyclohexen enthalten.

Um die inhibierende Wirkung des Luftsauerstoffs auszuschliessen setzt man photohärtbaren Systemen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert. Auch durch Einführung von autooxydablen Gruppen, beispielsweise Allylgruppen, in das zu härtende Harz kann der Luftsauerstoff desaktiviert werden.

Die Photoinitiatoren können auch in Kombination mit radikalischen Initiatoren, wie z. B. Peroxiden, Hydroperoxiden, Ketonperoxiden oder Percarbonsäureestern, verwendet werden.

Photopolymerisierbare Systeme enthalten weiterhin — je nach Verwendungszweck — Füllstoffe wie Kieselsäure, Talkum oder Gips, Pigmente, Farbstoffe, Fasern, Thixotropiemittel oder Verlaufhilfsmittel.

Ferner können auch Kombinationen mit bekannten Photoinitiatoren, wie Benzoinäthern, Dialkoxyacetophenonen oder Benzilketalen, verwendet werden.

Besonders für die Photopolymerisation von dünnen Schichten und Druckfarben können Kombinationen der erfindungsgemässen Photoinitiatoren mit Aminen und/oder aromatischen Ketonen verwendet werden. Beispiele für Amine sind Triäthylamin, N-Methyldiäthanolamin, N-Dimethyläthanolamin oder p-Dimethylaminobenzoesäureester. Beispiele für Ketone sind Benzophenon, substituierte Benzophenonderivate, Michler's Keton, Anthrachinon und Anthrachinonderivate, Cumarinderivate, sowie Thioxanthon und dessen Derivate.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet sind die erfindungsgemässen Initiatoren auch für photohärtbare Systeme zur Herstellung von Druckplatten. Hierbei werden z. B. Gemische von löslichen linearen Polyamiden mit photopolymerisierbaren Monomeren, beispielsweise Acrylamiden, und einem Photoinitiator verwendet. Filme und Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der UV-Härtung ist die Metallbeschichtung, beispielsweise bei der Lackierung von Blechen für Tuben, Dosen oder Flaschenverschlüssen, sowie die UV-Härtung von Kunststoffbeschichtungen, beispielsweise von Fussboden- oder Wandbelägen auf PVC-Basis.

Beispiele für die UV-Härtung von Papierbeschichtungen sind die farblose Lackierung von Etiketten, Schallplatten-Hüllen oder Buchumschlägen.

Erfindungsgemäss können die Verbindungen der Formel I auch als Initiatoren zur photochemischen Vernetzung von Polyolefinen verwendet werden. Hierfür kommen z. B. Polypropylen, Polybutylen, Polyisobutylen sowie Copolymerisate wie z. B. Aethylen-Propylen-Copolymere in Frage, vorzugsweise jedoch Polyäthylen von niedriger, mittlerer oder hoher Dichte.

Die Photoinitiatoren werden für die angeführten Anwendungsgebiete zweckmässig in Mengen von 0,1 bis 20 Gew.-%, vorzugsweise etwa 0,5 bis 5 Gew.-%, bezogen auf die photopolymerisierbare bzw. vernetzbare Zusammensetzung, angewendet. Unter Zusammensetzung ist hierbei das Gemisch aus der photopolymerisierbaren bzw. vernetzbaren Verbindung, dem Photoinitiator und den sonstigen Füll- und Zusatzstoffen gemeint, wie es in der jeweiligen Anwendung verwendet wird.

Der Zusatz der Photoinitiatoren zu den photopolymerisierbaren Zusammensetzungen geschieht im allgemeinen durch einfaches Einrühren, da die meisten dieser Zusammensetzungen flüssig oder gut löslich sind. Meist kommt es zu einer Lösung der erfindungsgemässen Initiatoren, wodurch deren gleichmässige Verteilung sowie die Transparenz der Polymerisate gewährleistet ist.

Die Polymerisation erfolgt nach bekannten Methoden der Photopolymerisation durch Bestrahlung mit Licht, das reich an kurzwelliger Strahlung ist. Als Lichtquellen sind z. B. Quecksilbermitteldruck-, -hochdruck- und niederdruckstrahler, sowie superaktinische Leuchtstoffröhren geeignet, deren Emissionsmaxima im Bereich zwischen 250 und 400 nm liegen.

Die erfindungsgemässen Propiophenone der Formel I besitzen eine verbesserte Reaktivität und Lagerbeständigkeit sowie einen erhöhten Widerstand gegen Vergilbung.

Die Herstellung und Verwendung der erfindungsgemässen Photoinitiatoren ist in den folgenden Beispielen näher beschrieben. Hierin bedeuten Teile Gewichtsteile und Prozente Gewichtsprozente.

| | | |
|---|---|---|
| 8 | 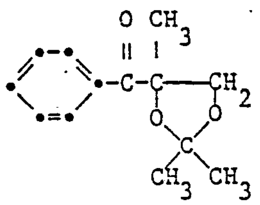 | (Oel) |

Beispiel 1 : Herstellung von 4-Benzoyl-2,2,4-trimethyl-1,3-dioxolan

16,2 g (0,1 Mol) 2-Benzoyl-2-methyl-oxiran werden in 35 g (0,6 Mol) Aceton gelöst, und unter Kühlen mit Hilfe eines Eisbads wird dann tropfenweise eine Lösung von 1,42 g (0,01 Mol) Bortrifluoridätherat in

35 g (0,6 Mol) Aceton zugegeben. Anschliessend wird während 2 Stunden bei Raumtemperatur gerührt und danach 5 Stunden unter Rückfluss erhitzt. Die Reaktionslösung wird abgekühlt, mit 50 ml gesättigter methanolischer Natriumhydroxid-Lösung unter Kühlung alkalisch gestellt und mit 200 ml Wasser verdünnt. Das Produkt wird mit Aether extrahiert. Die Aether-Lösung wird mit Kaliumcarbonat getrocknet und eingeengt. Der Rückstand wird im Vakuum bei 95 °C und 0,5 mbar destilliert. Das Destillat wird durch Mitteldruckchromatographie gereinigt, wobei ein farbloses Oel resultiert.

Analytische Daten:
Verbrennungsanalyse für $C_{13}H_{16}O_3$
Berechnet: C % 70,89 H % 7,33
Gefunden: C % 70,76 H % 7,31

$^1$H-NMR-Spektrum [$CDCl_3$, δ (in ppm)]:
8,25-7,95 (M, 2H); 7,45-7,2 (M, 3H); 4,64 und 3,70 (AB-System, J = 16 Hz, 2H); 1,57 (S, 3H); 1,43 (S, 3H); 1,10 (S, 3H), wobei S Singulett und M Multiplett bedeutet.

Anwendungsbeispiele

Beispiel 2

Eine Harzmischung aus 50 Teilen Actylan® AJ20 (Acrylatharz der Fa. SNPE, Paris Cedex), 15 Teile DQM-672 (Acrylat-Monomer der Fa. Rohm und Haas, Philadelphia, USA), je 15 Teile Trismethylolpropantriacrylat und Hexandioldiacrylat (Mehrfunktionelle Monomere der Fa. Degussa, Frankfurt), 10 Teile N-Vinylpyrrolidon (Reaktivverdünner der Fa. GAF, New York, USA) und 2 Teile Photoinitiator wird mit einem Filmziehgerät in einer Dicke von 40 µm Trockenschichtstärke auf Glasplatten aufgezogen. Diese Filme werden ca. 20 Sekunden abgelüftet und anschliessend mit einem Hg-Hochdruckstrahler (UV-Processor Model 1202 AN der Fa. PPG, Radiation Polymer Company, USA) bestrahlt. Dabei werden die Proben auf einem Transportband mit einer solchen Bandgeschwindigkeit unter der UV-Lampe vorbeibewegt, dass sich eine effektive Belichtungszeit von 1,05 Sekunden pro Durchlauf ergibt.

In der folgenden Tabelle sind in der zweiten Spalte die Anzahl Durchläufe (D) angeführt, die notwendig waren, um klebfreie Filme zu erhalten (wischfest).

In der dritten Spalte wird die Härte der Filme nach angegebener Anzahl von Durchläufen, gemessen mit dem Pendelgerät nach König, angegeben.

Zur Beurteilung der Verfärbung (Vergilbung) wird der Yellowness-Index nach ASTM D 1925-70 bestimmt.

Tabelle

| Photoinitiator gemäss Beispiel | Nötige Durchläufe | Pendelhärte nach König bei wischfest (Sekunde) | Yellowness-Index |
|---|---|---|---|
| 1 | 6 | 153 | 10 |

## Patentansprüche

1. Verwendung Verbindungen der Formel I,

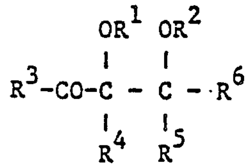

(I)

worin

$R^1$ und $R^2$ zusammen ein $C_3$-$C_6$ Alkyliden- oder ein mit Hydroxy, $C_1$-$C_4$ Alkoxy oder Phenyl substituiertes $C_2$-$C_6$-Alkyliden-, ein geradkettiges oder verzweigtes $C_2$-$C_6$ Alkadiyl-, ein Cyclopentyliden-, Cyclohexyliden-, ein 2,2,2-Trichloräthyliden-, 2-Furylmethyliden- oder Dimethylsilyliden-Radikal darstellen,

$R^3$ unsubstituiertes oder durch einen oder mehrere Reste —Cl, $C_1$-$C_4$ Alkyl, $C_1$-$C_4$ Alkoxy oder $C_1$-$C_4$ Alkylthio substituiertes Phenyl, ferner Benzoylphenyl, Phenoxyphenyl oder Phenylthiophenyl,

$R^4$ $C_1$-$C_4$ Alkyl,

und

$R^5$ Wasserstoff oder $C_1$-$C_4$ Alkyl bedeuten, oder ferner $R^4$ und $R^5$ zusammen ein Tri- oder Tetramethylen-Radikal darstellen, und

$R^6$ Wasserstoff, $C_1$-$C_4$ Alkyl, —$CCl_3$ oder Phenyl darstellt,

als Initiatoren für die Photopolymerisation äthylenisch ungesättigter Verbindungen sowie für die photochemische Vernetzung von Polyolefinen.

2. Photopolymerisierbare Zusammensetzung enthaltend mindestens eine äthylenisch ungesättigte photopolymerisierbare Verbindung und 0,1 bis 20 Gew.-%, bezogen auf das Gewicht der Zusammensetzung, einer Verbindung der Formel I des Anspruchs 1 als Photoinitiator sowie gegebenenfalls weitere bekannte und übliche Zusatzstoffe.

3. Photopolymerisierbare Zusammensetzung gemäss Anspruch 2, dadurch gekennzeichnet, dass sie als äthylenisch ungesättigte photopolymerisierbare Verbindung einen Mono-, Di- oder polyfunktionellen Acrylsäureester oder Methacrylsäureester oder Mischungen davon enthält.

4. Druckfarben enthaltend eine photopolymerisierbare Zusammensetzung gemäss Anspruch 2.

5. Metall-, Kunststoff- und Papierbeschichtungen enthaltend eine photopolymerisierbare Zusammensetzung gemäss Anspruch 2.

6. Verbindungen der Formel I des Anspruchs 1, worin $R^1$ und $R^2$ zusammen ein $C_3$-$C_6$ Alkyliden- oder ein mit Hydroxy, $C_1$-$C_4$ Alkoxy oder Phenyl substituiertes $C_2$-$C_6$-Alkyliden-, ein geradkettiges oder verzweigtes $C_2$-$C_6$ Alkadiyl-, ein Cyclopentyliden-, Cyclohexyliden-, ein 2,2,2-Trichloräthyliden-, 2-Furylmethyliden- oder Dimethylsilyliden-Radikal,

$R^3$ unsubstituiertes oder durch einen oder mehrere Reste —Cl, $C_1$-$C_4$ Alkyl, $C_1$-$C_4$ Alkoxy oder $C_1$-$C_4$ Alkylthio substituiertes Phenyl, ferner Benzoylphenyl, Phenoxyphenyl oder Phenylthiophenyl,

$R^4$ $C_1$-$C_4$ Alkyl,

und

$R^5$ Wasserstoff oder $C_1$-$C_4$ Alkyl bedeuten, oder ferner $R^4$ und $R^5$ zusammen ein Tri- oder Tetramethylen-Radikal darstellen,

und

$R^6$ Wasserstoff, $C_1$-$C_4$ Alkyl, —$CCl_3$ oder Phenyl darstellt.

7. Verbindungen der Formel I des Anspruchs 1 gemäss Anspruch 6, worin $R^1$ und $R^2$ zusammen Isopropyliden oder Dimethylsilyliden, $R^3$ Phenyl, $R^4$ Methyl, $R^5$ Wasserstoff oder $R^4$ und $R^5$ zusammen ein Tetramethylen-Radikal und $R^6$ Wasserstoff oder Phenyl bedeuten.

8. 4-Benzoyl-2,2,4-trimethyl-1,3-dioxolan.

## Claims

1. The use of a compound of the formula I

$$\begin{array}{ccccc} & & OR^1 & & OR^2 & \\ & & | & & | & \\ R^3\text{-}CO\text{-} & & C & - & C & -R^6 \\ & & | & & | & \\ & & R^4 & & R^5 & \end{array} \qquad (I)$$

in which

$R^1$ or $R^1$ and $R^2$ together are a $C_3$-$C_6$ alkylidene radical or a $C_2$-$C_6$ alkylidene radical which is substituted by hydroxyl, $C_1$-$C_4$ alkoxy or phenyl, a linear or branched $C_2$-$C_6$ alkadiyl radical, a cyclopentylidene or cyclohexylidene radical or a 2,2,2-trichloroethylidene, 2-furylmethylidene or dimethylsilylidene radical,

$R^3$ is phenyl which is unsubstituted or substituted by one or more —Cl, $C_1$-$C_4$ alkyl, $C_1$-$C_4$ alkoxy or $C_1$-$C_4$ alkylthio radicals, and is also benzoylphenyl, phenoxyphenyl or phenylthiophenyl,

$R^4$ is $C_1$-$C_4$ alkyl,

and

$R^5$ is hydrogen or $C_1$-$C_4$ alkyl, or $R^4$ and $R^5$ together are a trimethylene or tetramethylene radical,

and

$R^6$ is hydrogen, $C_1$-$C_4$ alkyl, —$CCl_3$ or phenyl,

as initiators for the photopolymerisation of ethylenically unsaturated compounds and for the photochemical crosslinking of polyolefines.

2. A photopolymerisable composition containing at least one ethylenically unsaturated photopolymerisable compound and 0.1 to 20 % by weight, based on the weight of the composition, of a compound of the formula I of claim 1 as a photoinitiator and also, if appropriate, further known and customary additives.

3. A photopolymerisable composition according to claim 2, which contains, as the ethylenically unsaturated photopolymerisable compound, a monofunctional, bifunctional or polyfunctional acrylic acid ester or methacrylic acid ester or mixtures thereof.

4. A printing ink containing a photopolymerisable composition according to claim 2.

5. Metal, plastics and paper coatings containing a photopolymerisable composition according to claim 2.

6. A compound of the formula I, of claim 1, in which

$R^1$ and $R^2$ together are a $C_3$-$C_6$ alkylidene radical or a $C_2$-$C_6$ alkylidene radical which is substituted by hydroxyl, $C_1$-$C_4$ alkoxy or phenyl, a linear or branched $C_2$-$C_6$ alkadiyl radical, a cyclopentylidene or cyclohexylidene radical or a 2,2,2-trichloroethylidene, 2-furylmethylidene or dimethylsilylidene radical,

$R^3$ is phenyl which is unsubstituted or substituted by one or more —Cl, $C_1$-$C_4$ alkyl, $C_1$-$C_4$ alkoxy or $C_1$-$C_4$ alkylthio radicals, and is also benzoylphenyl, phenoxyphenyl or phenylthiophenyl,

$R^4$ is $C_1$-$C_4$ alkyl,

and

$R^5$ is hydrogen or $C_1$-$C_4$ alkyl, or $R^4$ and $R^5$ together are a trimethylene or tetramethylene radical,

and

$R^6$ is hydrogen, $C_1$-$C_4$ alkyl, —$CCl_3$ or phenyl.

7. A compound of the formula I of claim 1, according to claim 6, in which $R^1$ and $R^2$ together are isopropylidene or dimethylsilylidene, $R^3$ is phenyl, $R^4$ is methyl, $R^5$ is hydrogen or $R^4$ and $R^5$ together are a tetramethylene radical and $R^6$ is hydrogen or phenyl.

8. 4-benzoyl-2,2,4-trimethyl-1,3-dioxolane.

**Revendications**

1. Application de composés répondant à la formule I :

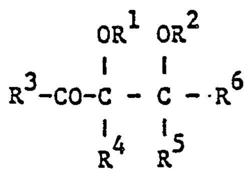

(I)

dans laquelle

$R^1$ et $R^2$ représentent ensemble un alkylidène en $C_3$-$C_6$, un alkylidène en $C_2$-$C_6$ porteur d'un hydroxy, d'un alcoxy en $C_1$-$C_4$ ou d'un phényle, un alcane-diyle en $C_2$-$C_6$ linéaire ou ramifié, un cyclopentylidène, un cyclohexylidène, un trichloro-2,2,2 éthylidène, un (furyl-2)-méthylidène ou un diméthyl-silylidène,

$R^3$ représente un phényle non substitué ou porteur d'un ou plusieurs substituants pris dans l'ensemble constitué par Cl, les alkyles en $C_1$-$C_4$, les alcoxy en $C_1$-$C_4$ et les alkylthio en $C_1$-$C_4$, également un benzoylphényle, un phénoxyphényle ou un phénylthio-phényle,

$R^4$ représente un alkyle en $C_1$-$C_4$ et

$R^5$ l'hydrogène ou un alkyle en $C_1$-$C_4$, ou encore $R^4$ et $R^5$ forment ensemble un radical triméthylène ou tétraméthylène,

et

$R^6$ représente l'hydrogène, un alkyle en $C_1$-$C_4$, —$CCl_3$ ou un phényle,

comme photo-amorceurs pour la photopolymérisation de composés éthyléniques ainsi que pour la réticulation photochimique de polyoléfines.

2. Composition photopolymérisable qui contient au moins un composé éthylénique photopolymérisable et, comme photo-amorceur, de 0,1 à 20 % en poids, par rapport au poids de la composition, d'un composé de formule I selon la revendication 1, ainsi que, le cas échéant, d'autres additifs connus et usuels.

3. Composition photopolymérisable selon la revendication 2, caractérisée en ce qu'elle contient, comme composé éthylénique photopolymérisable, un ester acrylique ou un ester méthacrylique mono-, di- ou polyfonctionnel, ou un mélange de tels esters.

4. Encres d'imprimerie qui contiennent une composition photopolymérisable selon la revendication 2.

5. Revêtements pour métaux, matières plastiques et papier qui contiennent une composition photopolymérisable selon la revendication 2.

6. Composés de formule I selon la revendication 1 dans lesquels :

$R^1$ et $R^2$ représentent ensemble un alkylidène en $C_3$-$C_6$, un alkylidène en $C_2$-$C_6$ porteur d'un hydroxy, d'un alcoxy en $C_1$-$C_4$ ou d'un phényle, un alcane-diyle en $C_2$-$C_6$ linéaire ou ramifié, un cyclopentylidène, un cyclohexylidène, un trichloro-2,2,2 éthylidène, un (furyl-2)-méthylidène ou un diméthyl-silylidène,

$R^3$ représente un phényle non substitué ou porteur d'un ou plusieurs substituants pris dans l'ensemble constitué par Cl, les alkyles en $C_1$-$C_4$, les alcoxy en $C_1$-$C_4$ et les alkylthio en $C_1$-$C_4$, également un benzoylphényle, un phénoxyphényle ou un phénylthio-phényle,

$R^4$ représente un alkyle en $C_1$-$C_4$ et

$R^5$ l'hydrogène ou un alkyle en $C_1$-$C_4$, ou encore $R^4$ et $R^5$ forment ensemble un radical triméthylène ou tétraméthylène,

et

$R^6$ représente l'hydrogène, un alkyle en $C_1$-$C_4$, $-CCl_3$ ou un phényle.

7. Composés selon la revendication 6, qui répondent à la formule I définie à la revendication 1, composés dans lesquels $R^1$ et $R^2$ forment ensemble un radical isopropylidène ou diméthylsilylidène, $R^3$ représente un phényle, $R^4$ un méthyle, $R^5$ l'hydrogène ou $R^4$ et $R^5$ forment ensemble un radical tétraméthylène, et $R^6$ représente l'hydrogène ou un phényle.

8. Benzoyl-4 triméthyl-2,2,4 trioxolanne-1,3.